# EUROPEAN PATENT SPECIFICATION

(11) **EP 0 493 575 B1**
(45) Date of publication and mention of the grant of the patent: **16.09.1998**
(21) Application number: 91913702.6
(22) Date of filing: 25.07.1991
(51) Int. Cl.: C12N 7/04, C12N 15/46, A61K 39/15

(54) **ROTAVIRUS REASSORTANT VACCINE**
IMPFSTOFF AUS REASSORTIERTEM ROTAVIRUS
VACCIN PRODUIT PAR REASSORTIMENT DE ROTAVIRUS

(30) Priority: 26.07.1990 US 558884
(43) Date of publication of application: 08.07.1992
(73) Proprietor: THE WISTAR INSTITUTE OF ANATOMY & BIOLOGY, Philadelpia, PA 19104-4268 (US); THE CHILDREN'S HOSPITAL OF PHILADELPHIA, Philadelphia, Pennsylvania 19104 (US)
(72) Inventor: PLOTKIN, Stanley, A., F-75016 Paris (FR); CLARK, H., Fred, Philadelphia, PA 19130 (US); OFFIT, Paul, Philadelphia, Pennsylvannia 19146 (US)
(74) Representative: Hale, Stephen Geoffrey
(86) International application number: US9105278
(87) International publication number: WO9201784

(56) References cited:
- EP-A- 0 323 708
- US-A- 4 571 385
- US-A- 4 636 385
- JOURNAL OF VIROLOGY, Volume 60, No. 2, issued November 1986, OFFIT et al., Reassortant rotaviruses containing structural Proteins vp3 and vp7 from Different Parents Induce Antibodies Protective against Each Parental Serotype", pages 491-496
- Modern Approaches to New Vaccines, issued 1988, KAPIKIAN et al., "Development of a Rotavirus vaccine by a "Jennerian" and a modified "Jennerian" approach", page 29
- PLOTKIN et al., editors, "Vaccines" published 1988 by W.B. Saunders (Philadelphia), see pages 517-525
- Third NTH Rotavirus Workshop, Summary issued 1988, HOSHINO, "Genetic Studies of Rotavirus Virulence," pages 50-51
- IMMUNIZATION MONITOR, Volume 3, No. 3, issued August 1989, CLARK, "Approaches to Immune Protection Against Rotavirus Diarrhea of Infants," pages 1-16
- VACCINE, Vol. 8, No. 4, issued 1990, CLARK et al., "Serotype 1 reassortant of Bovine Rotavirus WC3 strain WT79-9 induces a polytypic antibody response in infants" pages 327-332
- JOURNAL OF INFECTIOUS DISEASE, Vol. 161, No. 6, issued June 1990, CLARK et al., "Immune Protection of Infants Against Rotavirus Gastroenteritis by a serotype1 reassortant of Bovine Rotavirus WC3", pages 1099-1104
- JOURNAL OF VIROLOGY, Vol. 64, No. 6, issued June 1990, WARD et al., "Serumneutralizing Antibody to vp4 and vp7 proteins in infants following vaccination with WC3 bovine rotavirus", pages 2687-2691

## Description

The present invention refers generally to novel rotavirus reassortants, vaccines employing the novel reassortants and methods for their preraration. More particularly, the invention provides a reassortant which contains both the gene encoding the v.p.7 neutralization antigen of a selected human rotavirus and the gene encoding the v.p.4 neutralization antigen of a selected human rotavirus.

### Background of the Invention

Rotaviruses are the single most important etiologic agent of infectious gastroenteritis (diarrhea), which is the leading cause of infant death in the world. Of the estimated 5 to 10 million infant deaths caused by acute infectious gastroenteritis yearly [Walsh et al, New Eng. J. Med., 301:967 (1979)], rotaviruses cause between 10 and 40% of the total deaths [deZoysa and Feachem, Bull WHO 63:569 (1985)]. Rotavirus-induced infectious gastroenteritis is one of the ten leading causes of infant death, even in developed nations [Ho et al, 27th Interscience Conf. Antimicrobiol Agents Chemotherapy, p2 (1987)].

Rotaviruses of primate and bovine origin are spherical viruses, about 70 nm in diameter and characterized by a double capsid structure. The rotavirus genome has eleven segments of doublestranded RNA and an RNA polymerase. Each segment of RNA is a gene that codes for a single protein gene product.

A majority of the presently identified animal and human rotaviruses are designated as Group A rotaviruses, and share common cross-reactive antigens [Estes et al, Immunochemistry of Viruses, Elsevier, Amsterdam:389 (1984)]. Different species of rotaviruses are distinguishable by distinct serotype-specific virus surface antigens, which are most easily detected in conventional serum-neutralization (SN) tests. In a SN test, an antiserum prepared against a purified virus of a specific serotype scores a higher SN titer with a virus of a homologous serotype than with a virus of a heterologous serotype. Among human rotaviruses, at least six serotypes are now recognized--serotypes 1, 2, 3, 4, 8 and 9 [Wyatt et al, Infect and Immun., 37:110 (1983); Matsuno et al, J. Virol., 54:623 (1985); and Clark et al, J. Clin. Microbiol., 25:1757 (1987)].

Presently available knowledge on the crossimmunity of various serotypes in animals and human has provided contradictory results with regard to the necessity for serotype specific antigenic stimulation to provide immune protection against rotavirus infection. See, for example, several reports on vaccine challenge studies in animals [Wyatt et al, Science, 203:548 (1979); Zissis et al, J. Inf. Dis., 148:1061 (1983); Sheridan et al, J. Inf. Dis., 149:434 (1984)]. See, also, several reports on rotavirus vaccines evaluated in animals and humans [Mebus et al, J.A.V.M.A., 163:880 (1973); Thurber et al, Canad. Vet. J., 17:197 (1976); Vesikari et al, Lancet, 2:807 (1983); De Mol et al, Lancet, 2:108 (1986); Clark et al, Amer. J. Dis. Children, 140:350 (1986); Kapikian et al, Vaccines, New York, Cold Spring Harbor Lab., 357 (1985); Losonsky et al, Ped. Inf. Dis., 5:25 (1986); and Santosham et al, 27th Int. Conf.
Antimicrobiol. Agents and Chemotherapy, p. 99 (1987)].

Efforts to develop an effective rotavirus vaccine have proven disappointing to date [H F. Clerk, "Rotavirus Vaccines," Vaccines, Plotkin S.A., Mortimer E.A., eds. (Philadelphia, W.B. Saunders: 1988), p. 517; H F. Clerk, Immunization Monitor, 3:3 (1989); H F. Clark et al, "New Vaccines Against Rotavirus Gastroenteritis," Advances in Pediatric Infectious Diseases (1990) in press]. See, also, "WHO News and Activities," Bull WHO, 67(5): 583-587 (1989), reporting on the efficacy of rotavirus vaccines.

Human rotaviruses have not been avidly pursued for vaccine use because cell culture-adapted human rotaviruses replicate inefficiently, particularly in cells considered to be acceptable as human vaccine substrates. Additionally, the potential pathogenicity of human rotavirus isolates is largely unknown.

Animal-origin vaccines have also proved unsatisfactory, when evaluated in clinical trials involving orally administered living virus. For example, the immunization of infants with bovine rotavirus vaccine strain RIT4237 has proved safe and induced measurable serum antibody response in 60-80% of recipients. Despite this initial promise, however, the RIT4237 vaccine has subsequently shown to provide inadequate protection against rotavirus disease.

Also tested in clinical trials as a candidate for human vaccine was simian-origin rotavirus RRV (MMU 18006). RRV was found to be more immunogenic than RIT4237 but also provided inconsistent protection against rotavirus disease. [G.A. Losonsky et al, Pediatr. Inf. Dis., 5:25 (1986); and T. Vesikari et al, J. Infect. Dis., 153: 832 (1986)].

A bovine-origin rotavirus of low-tissue culture passage level, strain WC3, which is safe in infants and causes a serum antibody response in 70-95% of recipients, has likewise been shown to provide inconsistent and limited protection [Plotkin et al, J. Infect. Dis., 159: 860-965 (1989)].

The ability to produce serum antibody is not a characteristic which necessarily provides a good vaccine candidate. The failure of such animal-origin vaccines to provide consistent protection has led to interest in vaccines incorporating genes from human rotaviruses coding for human type-specific antigens. This incorporation, called "reassortment", is possible because of the segmented nature of the RNA genome of rotavirus and its high frequency of gene reassortment during coinfection. See, e.g., U.S. Patent No. 4,571,385 which describes a method of producing a rotavirus reassortant from human and animal parental strains by combining the human rotavirus with a cultivatable animal rotavirus and selecting for desired reassortants with an antibody specific for the 34-38 kd glycoprotein, v.p.7 (gene 8 or 9) of the animal virus.

In rotavirus reassortants of bovine\simian origins, it was observed that the v.p.7 and v.p.4 major outer capsid proteins (each from a different parent) were independently capable of inducing a protective immune response in mice against virulent rotavirus challenge when administered at high dosages [Offit et al, J. Virol., 60(2):491-496 (1986); Offit et al, J. Virol., 57 (2) :376-378 (1986)].

Previously known reassortant rotaviruses proposed for use as human vaccine candidates involved the replacement of a single gene product encoding the v.p.7 antigen of an animal rotavirus. The 38kd v.p.7 antigen of gene 9 or 8 of the animal virus, has been replaced with the v.p.7 encoding gene of a human serotype rotavirus. Such reassortants containing the human v.p.7 encoding gene include reassortants based upon U. K. strain bovine rotavirus with a v.p.7 of human serotype 1, 2, 3 or 4; or based upon simian RRV rotavirus with the gene coding for serotype-specific surface antigen v.p.7 supplied from human serotype 1, 2, or 4 [Midthun et al, J. Virol., 53:949 (1985); Midthun et al, J. Clin. Microbiol., 24:822 (1986) and United States Patent 4,571,385].

Other reassortants have been formed by coinfection of two human strains, but have never been tested for vaccine use. [See, e.g., Urasawa et al., J. Gen. Virol., 67:1551-1559 (1987)].

The use of reassortant rotavirus vaccine containing v.p.4 of human rotavirus origin, however, has been avoided because of its suspected role in virulence and the difficulty in propagating human v.p.4-containing reassortants in conventional tissue culture [P.A. Offit et al, J. Virol., 57:46 (1986)]. More recently it has been postulated that virulence is polygenic [D. Chen et al, Proc. Natl. Acad. Sci. USA, 86:3743 (1989); Y. Hoshino, "Genetic studies of rotavirus virulence", Summary, third NIH Rotavirus Workshop, Bethesda, MD: 50 (1988)].

Like the animal rotavirus vaccine candidates, the human/animal reassortants have been found to elicit an increased, but disappointing, incidence of human serotype-specific responses. Additionally, RRV reassortant derivatives have induced undesirable side effects (fever, gastrointestinal symptoms) in a significant proportion of vaccines in clinical trials [Vesikari et al, cited above; and Losonsky et al, cited above]. Thus,like the animal vaccines, the ability of a reassortant to induce neutralizing antisera is not itself indicative of the usefulness of the reassortant as an effective and safe vaccine for humans.

What is needed in the art is an effective rotavirus vaccine, i.e. one capable of providing long-lasting protection against rotavirus infection, especially in human infants and neonates.
We have found that novel reassortants containing from a selected human rotavirus at least the human rotavirus v.p.7-encoding gene segment and the human rotavirus v.p.4-encoding gene segment, with the non-human gene segments from a bovine rotavirus strain noted to be safe in humans, can be used as components of vaccines against human rotavirus infection capable of inducing a protective immune response in humans.

According to one aspect of the invention there is provided a rotavirus reassortant useful as a component of a vaccine against human rotavirus infection, said reassortant comprising gene 4 from a human rotavirus encoding the v.p.4 neutralization antigen and the gene from a human rotavirus encoding the v.p.7 neutralization antigen, said reassortant characterized by remaining rotavirus segments originating solely from a bovine WC3 strain rotavirus or progeny of said strain substantially identical thereto or from both said human and said bovine strains, and said reassortant further characterized as capable of inducing a protective immune response in human infant and child patients to challenge with native rotavirus without producing serious adverse effects in said patients.

As another aspect of this invention there is provided a vaccine for providing immunological protection against acute diarrhea caused by human rotavirus, said vaccine comprising at least one rotavirus reassortant comprising gene 4 from a human rotavirus encoding the v.p.4 neutralization antigen and the gene from a human rotavirus encoding the v.p.7 neutralization antigen, said reassortant characterized by remaining rotavirus segments originating solely from a bovine WC3 strain rotavirus or progeny of said strain substantially identical thereto or from both said human and said bovine strains, and said reassortant further characterized as capable of inducing a protective immune response in human infant and child patients to challenge with native rotavirus without producing serious adverse effects in said patients.

Another aspect of the invention is a method for preparing a novel reassortant virus of the invention. This method involves infecting a suitable cell substrate with a mixed infection of a bovine rotavirus strain WC3 and a human rotavirus under conditions enabling gene reassortment in the infected culture. Progeny clones from plaques formed in the infected cell substrate (culture) are examined by polyacrylamide gel electrophoresis (PAGE) for the presence of a reassortant containing a human rotavirus gene segment encoding the v.p.4 neutralization antigen and a human rotavirus gene segment encoding the v.p.7 neutralization antigen with the remaining rotavirus segments contributed solely by the bovine strain (safe for use in humans) or progeny of said strain substantially identical thereto or by both the human and the bovine strains.

The vaccines of the present invention may be used to vaccinate humans against human rotavirus infection.

This vaccination program may also employ more than one of the vaccine compositions of this invention optionally with reassortants in which only one of the two gene segments derived from human rotavirus and the other is derived from bovine WC3 rotavirus.

For example, a patient may be serially or simultaneously vaccinated with vaccine compositions containing the v.p. 4 gene from different human rotavirus serotypes. This method involves administering by oral or nasal route, or by injection, to human beings at least one dose comprising from about 10^{6.0} to about 10^{9.0} pfu, of the vaccine. This method can also employ an additional dose of the vaccine about 3 to 4 weeks after the first dose. The vaccine may be administered directly to infants or to nursing mothers for purposes of transferring immunity to an infant. The vaccine may also be administered annually to infants or children prior to the annual rotavirus season.

Other aspects and advantages of the present invention will be apparent upon consideration of the following detailed description of the invention, including illustrative examples of the practice thereof.

### Detailed Description of the Invention

This invention involves rotavirus reassortants suitable for use as vaccines, which are characterized by safety to humans and the ability to confer immune protection against human rotavirus infection. The reassortants are produced by genetic reassortment between an attenuated bovine WC-3 strain rotavirus (or progeny of said strain substantially identical thereto and a rotavirus representing an epidemiologically important human serotype. The human rotavirus contributes to the novel reassortant at least the gene segment encoding the v.p.4 protein and the gene segment encoding the v.p.7 protein.

As will be indicated below, the vaccination program may also include, as an additional component to the use of the novel rotavirus reassortant of the present invention, a rotavirus reassortant in which either but not both of the gene segments encoding the v.p.4 and v.p.7 proteins are contributed by the human rotavirus, the other of those segments being contributed by the bovine WC3 rotavirus strain and the remaining gene segments by either the human or the bovine WC3 strains.

The human rotavirus genes which encode for the neutralization antigens v.p.7 and v.p.4 in the novel reassortant may be selected from any human rotavirus serotype for which immunization is desired. A non-exclusive list of such serotypes includes the serotype 1, serotype 2, serotype 3, serotype 4, serotype 8 and serotype 9, as well as new viral serotypes yet to be identified.

Among the human rotavirus strains useful in the present invention are strains WI79, WICC1, WI/U of serotype 1; strains WI-SC2 and WI/Q of serotype 2; strains WI77, WI78, WI/P, and WIC-17 of serotype 3; strain WI-CC4 of serotype 4; and strain WI61 of serotype 9. This list of human serotype rotaviruses is non-exclusive; newly identified human origin rotaviruses are also expected to be useful in the methods and compositions disclosed herein.

The protein v.p.4 is an 88,000 dalton major surface structural protein product of gene 4 of a rotavirus. Like v.p.7, it functions as a major serotype-specific antigen, operative in SN tests, capable of inducing serotype-specific neutralizing antibody, and capable in a mouse system of inducing serotype specific immune protection against rotavirus disease. [See, Offit et al, (1986) supra]. In experimental studies bovine v.p.4 has been shown to also play a role in the control of rotavirus host range in cell culture [Kalica et al, Virol , 125:194 (1983); Greenberg et al, Infec. Immunol., 37:104 (1982)], and in the mediation of rotavirus virulence in vivo [Offit et al, J. virol., 57:46 (1986)]. Reassortants containing human v.p.4 antigen have been considered difficult to cultivate in tissue culture media [Greenburg, cited above].

Without being bound by theory, nucleic acid sequences of gene 4 and antigenic analyses of v.p.4 suggest that there are only three basic classes of human v.p.4. The classes consist respectively of (1) v.p.4 of types 1, 3, 4 and 9; (2) v.p.4 of types 2 and 8; and (3) v.p.4 of "nursery strains" [O. Nakagomi et al, Molecular and Cellular Probes, 3:251 (1989)]. Because the majority of human infections are caused by serotypes 1, 2, 3 and 4, immunization with two v.p.4 antigens, one from each group (1) and (2), would provide optimal coverage of the v.p.4 specificities of most natural infections.

Thus, the rotavirus contributing the v.p.4 encoding gene segment may be from any selected human serotype virus and more than one strain may be used in the vaccination of an individual patient.

The v.p.7 protein is coded for by either gene segment 7, gene segment 8 or gene segment 9 of the particular human rotavirus. The location of the v.p.7 encoding gene may be determined for each specific rotavirus by conventional experimental methods. The rotavirus contributing the v.p.7 protein-encoding gene segment may be from any selected human serotype virus, as identified above. The selected human rotavirus may also be attenuated, if desired, for use in the reassortant.

A reassortant according to the present invention contains both human rotavirus gene 4, encoding v.p.4 and the human rotavirus gene 7, 8, or 9 encoding v.p.7. The human rotavirus may desirably contribute more than the v.p.7 and v.p.4 encoding genes to the reassortant.

The remaining gene segments of the novel reassortants are obtained from the attenuated bovine rotavirus strain WC3 or its progeny, described in detail in United States Patent 4,636,385, or from both said human and said bovine strains. The disclosures of that patent are incorporated by reference herein to provide additional information about this rotavirus strain. WC3 replicates to a high titer in CV-1 cells (ATCC CCL70) and in Vero cells (ATCC CCL-81) and is known to be attenuated and immunogenic in human infants [Clark et al, Amer. J. Dis. Children, 140:350 (1986)].

The reassortants provided by the present invention contain the gene encoding the v.p.4 protein contributed by the selected human rotavirus. For a reassortant for use as an additional component of the vaccination program the gene encoding the v.p.7 protein may be contributed by attenuated bovine rotavirus WC3. However, for the novel reassortants of the invention this gene and the gene encoding the v.p.7 protein must both be contributed by the human rotavirus. As identified below, the reassortants strain contributing the fewest segments to the reassortant is identified with the segment numbers occuring after the dash.

The novel reassortants contain both the human v.p.4 and human v.p.7 encoding gene segments. WI79-4,9, which is an example of such a novel reassortants contains both genes 4 and 9 from the human strain WI79 and genes 1 to 3, 5 through 8, and 10 to 11 from bovine strain WC3. An example of a reassortant useful as an additional component of the vaccination program is WI79-4, which contains gene 4 encoding the v.p.4 protein from strain WI79 and genes 1 to 3, and 5 through 11 from bovine strain WC3. Another such reassortant is WI78-4, which contains gene 4 from human strain WI78 and the remaining ten genes from WC3. Reassortant WI61-4 similarly contains gene 4 from the human serotype 9 rotavirus strain WI61, and the remaining genes from WC3. Reassortant WISC2-4 contains gene 4 from the serotype 2 human virus SC2 and the remaining genes from the bovine strain WC3.

Other reassortants useful as additional components of the vaccination program are reassortants containing the v.p.7 antigen from the human rotavirus. A specific embodiment is reassortant WI79-9, containing gene segment 9 encoding v.p.7 from human rotavirus WI79, with gene segments 1-8, 10 and 11 from WC3.

Another useful reassortant of this type, designated WC3:2-5, contains the v.p. 7 encoding gene segment from a human serotype 3 rotavirus. Specifically in this reassortant four genes, 2 through 5, derive from bovine rotavirus WC3 and seven genes, 1, and 6 through 11 of human serotype 3 rotavirus strain WI78. Another useful reassortant carrying the human v.p.7 encoding gene segment is WC3:2-6, which contains five genes, 2 through 6, from bovine rotavirus WC3 and six genes, 1, and 7 through 11, from human serotype 3 rotavirus strain WI78. Reassortant WI78-8 contains the v.p.7 gene from human strain WI78 and all remaining gene segments from WC3.

Additional exemplary reassortants utilize human serotype WI61 as the contributor of the v.p.7 protein encoding gene. For example, WI61:7,9 contains genes 7 and 9 from human serotype 9 strain WI61 and genes 1 through 6, 8, 10 and 11 from WC3.

WI79-5 and WC3:2-5 have been deposited with the American Type Culture Collection, 12301 Parklaw Drive, Rockville, Maryland. WI79-9 was deposited on November 25, 1987 under accession number ATCC VR2194 and ATCC VR2196 and WC3:2-5 was deposited on November 25, 1987 under accession number ATCC VR2193 and ATCC VR2195. Unless stated to the contrary, all deposits with the ATCC referred to herein, are available to the public upon grant of a patent to the assignee, The Wistar Institute of Anatomy and Biology, Philadelphia, PA, USA.

All of the above-identified reassortants (with the exception of reassortants WISC-4 and WI78-4 which are in the process of preparation but which are fully described herein) were in existence at the time that either this application or its parent application SN 07\125,477 was filed. All reassortants are kept permanently in the laboratory of the inventor Dr. H Fred Clark, at the Childrens Hospital of the University of Pennsylvania, Philadelphia, PA, U.S.A. from the time they are produced. The inventors are willing to make the reassortants available to appropriate persons at the USPTO upon request during the pendency of this application and will make the reassortants available to the public without restriction after issuance of a patent containing appropriate claims.

Two of the reassortants used in the vaccination program are described in detail below. These reassortants, as well as the novel reassortants and other reassortants envisaged as additional components in the vaccination program, can be obtained by one skilled in the art by following the procedures disclosed herein.

The method for producing the reassortants includes the step of isolating the human and other species rotavirus by culturing in a suitable cell culture. Briefly, the two parent viruses are co-infected by conventional techniques and the progeny viruses are identified by running each parent and the progeny on conventional gel electrophoresis. Since each gene segment runs at a characteristic molecular weight, the makeup of the reassortant is easily identified by comparison with the parent. Performing the infections and gel electrophoresis techniques to obtain such reassortants are skills known to the art. The isolation technique is standard and is described in more detail in Example 1.

Suitable cells for such isolation and infection include primate VERO cells (ATCC CCL-81), African green monkey kidney cells CV-1 (ATCC CCL-70); BSC-1 (ATCC CCL-26), fetal green monkey cell MA-104, and primary primate kidney cell cultures. Vero cells are presently preferred. For purposes of this invention, primary primate kidney cell cultures include first, second (secondary) or third (tertiary) passages of kidney cells derived from the indicated species of primate. Each of these cell culture substrates may be grown in BHK medium [MacPherson, I and M. Stoker, Virology, 16:147 (1962)], supplemented with 10% fetal calf serum, Eagle's minimal essential medium with 10% fetal calf serum, or medium 199 with 10% fetal calf serum. These media may also contain gentamicin, 25 micrograms per milliliter. These cell lines may be used alone, or in combination in serial passaging of the viruses. When used in combination, a separate but different cell line can be used in each of the various passages of the virus.

Secondly, a suitable cell culture is infected with both the attenuated bovine rotavirus strain WC3 and the desired human serotype rotavirus. Mixed infections are designed to maximize the potential for reassortment by ensuring that large and equal concentrations of each parent virus are replicating simultaneously. After infection and sufficient time and conditions for gene reassortment, reassortant progeny clones are examined by random selection of plaques, e.g., by performing a plaque assay of the virus yield from the mixed infection. The virus is propagated in individual plaques which are induced by inoculation of the yield of the mixed infection onto another cell culture monolayer.

Polyacrylamide gel electrophoresis with silver stain (PAGE-SS) according to the procedure of Dolan et al, J. Clin. Microbiol., 21:753 (1985) is employed to analyze each such virus population and compare its electropherotype with that of each parental rotavirus. The proportion of reassortant rotaviruses isolated may be enhanced by selecting plaques whose morphology differs from that of either parent. Alternatively, progeny clones may be selected from the virus yield of the mixed infection after treatment with hyperimmune antiserum to the serotype of the rotavirus contributing the v.p.4 encoding gene [See, e.g., the method of United States Patent 4,571,385], prior to performing the plaque analysis of the population. This method may be applied to any human or animal virus.

Progeny clones are examined by harvesting individual plaques, which are then cultivated individually in cell culture and examined for their gene constitution by PAGE-SS. Reassortant progeny clones are selected as vaccine candidates if their PAGE-SS reveals the presence of at least the gene coding for the surface antigens v.p.4 and/or v.p.7 from the human rotavirus against which immune protection is being sought. Preferably, the PAGE-SS will reveal the presence of human v.p.4 in the reassortant or both the v.p.4 and v.p.7 encoding gene segments from the human rotavirus parental strain.

Vaccines for providing immunological protection against acute diarrhea caused by human rotavirus infection may contain one or more of the novel reassortants of the present invention. Optionally, the vaccines may also contain conventional vaccine adjuvants and/or carriers, e.g., aqueous suspensions of aluminum and magnesium hydroxides. The method of preparing a vaccine according to the invention involves inoculating a suitable cell substrate, e.g., Vero cells or CV-1 cells, and passaging the reassortant therein. By combining one or more different human serotype reassortants, the vaccine can elicit a polytypic viral neutralizing antibody response.

The vaccine preparations including one or more of the reassortants described herein are administered, preferably by oral or nasal route, in a suitable dose. The vaccine may also be administered by injection. Alternatively, the vaccine may be administered to nursing mothers as a means for transfering immunity to the infant.

The dosage for all routes of administration is generally greater than 10⁶, between 10⁶ and 10⁹ plaque forming units (pfu) of the reassortant, with the preferred dosage being 10⁷ pfu. Additional doses of the vaccines may also be administered. It may be preferable to inoculate susceptible infants and children on an annual basis prior to the "rotavirus season". Rotavirus infection in humans has been observed to occur in various geographical regions during the same season, e.g. in winter in the northeastern United States. Repeated inoculations prior to that season or susceptible infants and children may be indicated.

The preparation of a pharmaceutically acceptable vaccine, having due regard to pH, isotonicity, stability and the like, is within the skill of the art. Conventional adjuvants may also be employed in the vaccine composition, e.g., aluminium hydroxide gel. The dosage regimen involved in a method for vaccination will be determined considering various hosts and environmental factors, e.g. the age of the patient, time of administration and the geographical location and environment.

The following Example 1 demonstrates the isolation of the rotavirus for producing the reassortants of the present invention and related reassortants and Example 2 illustrates a specific novel rotavirus reassortant (WI79-4,9) and other reassortants useful as additional components of the vaccination program. Examples 3 and 4 illustrate methods for making and administering vaccines, although the vaccines are not in accordance with the invention in so far as they do not include novel reassortants according to the invention.

### Example 1 - Isolation of the Rotaviruses

The bovine rotavirus strain WC3 and human rotavirus strains used in producing reassortants according to the invention were isolated in cell line MA104 or in Vero cells and then adapted to growth in cell lines CV-1 or Vero.

The human origin rotaviruses were isolated by standard techniques as described previously for isolation of human rotavirus strain WI61 in Clark et al (1987) supra. Stools of infants ill with gastroenteritis were determined to contain rotavirus by means of examination by the PAGE-SS technique for detection of the rotavirus-characteristic 11 segments of double-stranded RNA. Rotavirus-containing stools were emulsified into a 5% (w/v) suspension in serum-free Eagle's Minimal Essential Medium containing 500 units of penicillin/ml, 500 micrograms of streptomycin/ml, 40 micrograms of gentamicin/ml 50 units of nystatin/ml, and 20 micrograms of trypsin/ml. The stool suspension was clarified by centrifugation at 2000Xg for 30 minutes.

Clarified supernatant fluid was incubated with an equal volume of purified trypsin (10 microgram/ml) in phosphate buffered saline (PBS) for 60 minutes at 37°C. The trypsin-treated stool supernatant fluid was inoculated in a volume of 0.2 ml into tube cultures of MA104 cells which had previously been washed three times with PBS. After absorption of this rotavirus-containing fluid for 30 minutes at 37°C, the tube cultures were fed with 1.5 ml of Sato medium containing 1 microgram/ml of purified trypsin and incubated in a roller apparatus at 37°C.

Inoculated cell cultures were harvested after seven days of incubation by freezing and thawing of the combined cells and cell culture medium. Serial passage was accomplished by inoculating 0.2 ml of undiluted cell culture suspension into fresh tubes of MA104 cell culture treated in the same manner as the initial passage inoculated with stool suspension supernatant fluid. Cell culture suspensions from each successive passage were analyzed for the presence of rotavirus RNA by the PAGE-SS technique. Detectable concentrations of rotavirus RNA were usually obtained by the second and third passage level. Visible cytopathic effect (CPE) usually appeared by the second to fifth cell culture passage.

After the rotavirus strain has become cytopathic, serial passages were made whenever CPE involved more than 75% of the cell monolayer (2 to 7 days). When a rotavirus isolate consistently induced CPE in roller tube cultures within 48 hours (usually within 4 to 8 passages), serial passage was performed in stationary cultures of MA104 cells fed with BHK medium supplemented with 13 micrograms/ml of unpurified trypsin (Flow Labs). Serial subculture in MA104 cell stationary cultures was performed in the same manner as that used for roller tubes, and was continued until the isolated rotavirus was determined to efficiently induce plaques under agarose overlay in MA104 cell culture.

When the rotavirus isolate efficiently induces plaques in the plaque induction assay according to Offit et al, J. Virol. Methods, 7:29 (1983) [usually 10⁵ to 10⁷ pfu per ml], it has adapted to growth in the MA104 cell culture. It is then adapted to growth in stationary cultures of CV-1 cells by similar serial passage methods, except that the medium is Eagle's MEM serum-free, containing 6.25 microgram/ml unpurified trypsin (Flow). At varying passage levels, as appropriate, the isolated rotavirus may be genetically purified by isolation and propagation of a single plaque produced in MA104 cell culture. Mechanical aspiration of cells within a single plaque, well separated from any surrounding plaques is followed by serial propagation of virus contained in this cell suspension by standard technique.

It is presently preferred to inoculate the virus into tube cultures of Vero cells in the presence of serum-free medium with 0.75 µg/ml purified trypsin. These cultures are incubated in roller apparatus at 37°C, with the trypsin being replenished at 3 to 4 day intervals. The culture is harvested by freezing and thawing about 7 to 10 days after inoculation. Subpassages are made in additional roller tubes of Vero cells or stationary cultures of Vero cells in tissue culture flasks. After 2-5 passages, virus is capable of causing CPE in stationary cultures, and may be used to prepare the vaccines.

The identity of the cell culture-adapted rotavirus compared with the virus in the original stool suspension is confirmed by comparison of the RNA electropherotypes induced in polyacrylamide gel. The serotype of each cell culture-adapted rotavirus may be determined by reaction with serotype-specific hyperimmune antisera to prototype rotaviruses prepared in rabbits and guinea pigs [Clark et al, (1987) supra].

### Example 2 - Producing the Reassortants

### A. WC3:2-5 and WI78-4

MA104 cell culture in a 24 well plastic plate (approximately 1 cm² of cell monolayer per well) was washed twice with PBS and inoculated with a mixture containing 1.5 X 10⁵ pfu of human serotype 3 rotavirus strain WI78 (passage level 17) and 1.5 X 10³ pfu of WC3 rotavirus (passage level 11). An excess of the WI78 was employed because WC3 grows more rapidly than most human-origin rotavirus isolates. The virus was allowed to absorb to the cells by incubation for 30 minutes at 37°C, after which 1.5 ml/well BHK medium with 13 micrograms/ml trypsin/ml was added and incubation was continued at the same temperature. When CPE involved the entire cell population at 72 hours post infection, the culture was harvested by three cycles of freezing and thawing and the progeny vIrus was plaqued by standard technique in MA104 cells. Plaques that were smaller than those induced by parental rotavirus WC3 were harvested, propagated, and analyzed by PAGE-SS.

Among the progeny plaques of this mixed infection was a reassortant designated WI78:1,7,9. This reassortant contained gene segments 1, 7 and 9 of parent rotavirus WI78 and all other gene segments from WC3. Another progeny plaque identified in this manner was WI78-4, containing the gene segment 4 (v.p.4) from WI78 and all remaining segments from WC3.

This virus was serially passaged 10 times in MA104 cells including three plaque purifications. Because WI78:1,7,9 was not neutralized by reference hyperimmune antiserum to serotype 3 rotavirus [see, e.g., Clark et al, AJDC, (1986) supra], it was observed that gene 8 of parent WI78, not gene 9, must code for the serotype 3-specific v.p.7 protein.

A second mixed infection was performed as above, with the WI78:1,7,9 reassortant mixed with WI78 rotavirus. Among the progeny plaques derived from this mixed infection was a reassortant which contained genes 2 through 5 from bovine rotavirus WC3 and all remaining genes from WI78 rotavirus.

This reassortant, designated WC3:2-5, was passaged six times in MA104 cell culture, including three plaque purifications. Reaction of WC3:2-5 virus in a virus neutralization (VN) test with hyperimmune reference sera to both human serotype 3 rotavirus and to bovine rotavirus serotype virus indicated that its phenotype is both serotype 3 and bovine.

WC3:2-5 also replicates to a titer of at least 10^{6.0} pfu/ml in CV-1 and Vero cells. At this dose, it is entirely attenuated for orally inoculated adults and for infants as young as two months of age. A high percentage of infants orally inoculated with WC3:2-5 respond, with approximately equal frequency, with VN antibody to human serotype 3 rotavirus and/or bovine serotype rotavirus.

### B. WI79-9, WI79-4 and WI79-4,9

MA104 cell culture in a 24 well plate was washed twice with PBS and inoculated with 0.2 ml of a suspension containing 2.0 X 10⁵ pfu of human serotype 1 strain WI79 rotavirus (WI79 was passaged eleven times in MA104 cells, including two plaque purifications, and thirteen times in CV-1 cells). This virus was allowed to absorb to cells for 60 minutes at 37°C, after which the virus was removed and the cells washed twice with PBS. 0.2 ml of a suspension containing 4.0 X 10¹ pfu of WC3 rotavirus (passage level 12) was added. The WC3 rotavirus was allowed to absorb for 60 minutes, after which the cells were washed three times with PBS and 1.5 ml of BHK medium with 13 micrograms/ml trypsin was added. Infected cells were incubated at 37° until CPE involved the entire monolayer (approximately 96 hours post infection).

The mixed infection was then harvested by three cycles of freezing and thawing. The cell culture fluids comprising this harvested infection were then reacted in a neutralization reaction consisting of addition of cell culture fluids to an equal volume of hyperimmune rabbit antiserum to bovine serotype rotavirus, obtained by conventional means and diluted 1:50. The resulting neutralization mixture was then incubated at 37°C for 30 minutes, after which the surviving virus was plaqued on MA104 cell culture by standard technique. Plaques induced in MA104 cell culture were harvested at random, propagated in MA104 cell culture, and analyzed by PAGE-SS for dsRNA electropherotype in comparison with parental rotaviruses WC3 and WI79.

Among these plaque isolates was a reassortant rotavirus, designated WI79-9, containing gene 9 from human serotype 1 WI79 and all other genes (1-8, 10 and 11) derived from bovine rotavirus strain WC3. Also identified in this manner was reassortant WI79-4, containing gene 4 from WI79 and remaining segments from WC3.

To generate reassortant WI79-4,9, which contains gene segments 4 and 9 (v.p.4 and v.p.7) from the human strain WI79, and remaining segments from WC3, reassortants WI79-9 and WI79-4 were combined in a culture and treated with anti-bovine rotavirus serum. WI79-4,9 was then simply harvested from the culture, since it contained neither the v.p.4 or v.p.7 antigens from the bovine strain.

WI79-9 is antigenically bivalent in VN tests with hyperimmune antisera. It reacts with antisera to bovine serotype and human serotype 1 rotaviruses. WI79-9 rotavirus replicates to a titer of 10^{7·0} pfu/ml in CV-1 cell culture. At this concentration, it is completely attenuated for orally inoculated adults and infants as young as two months of age. In a high percentage of infants, WI79-9 rotavirus induces VN antibody specific for rotavirus serotype 1 and/or the bovine rotavirus serotype.

WI79-4 is similarly neutralized by antisera to human type 1 rotavirus and by antisera to bovine rotavirus.

WI79-4,9 is not neutralized at all by bovine antisera, even though it contains nine bovine genes. It is neutralized by antisera to human type 1 rotavirus.

### C. WI61-4 and WI61-7,9

MA104 cell culture in a 24 well plate was washed twice with PBS and inoculated with 0.2 ml of a suspension containing 2.0 X 10⁵ pfu of human serotype 9 strain WI61 rotavirus (WI61 was passaged eleven times in MA104 cells, including two plaque purifications, and thirteen times in CV-1 cells). This virus was allowed to absorb to cells for 60 minutes at 37°C, after which the virus was removed and the cells washed twice with PBS. 0.2 ml of a suspension containing 4.0 X 10¹ pfu of WC3 rotavirus (passage level 12) was added. The WC3 rotavirus was allowed to absorb for 60 minutes, after which the cells were washed three times with PBS and 1.5 ml of BHK medium with 13 micrograms/ml trypsin was added. Infected cells were incubated at 37°C until CPE involved the entire monolayer (approximately 96 hours post infection).

The mixed infection was then harvested by three cycles of freezing and thawing. The cell culture fluids comprising this harvested infection were then reacted in a neutralization reaction consisting of addition of cell culture fluids to an equal volume of hyperimmune rabbit antiserum to bovine serotype rotavirus, obtained by conventional means and diluted 1:50. The resulting neutralization mixture was then incubated at 37°C for 30 minutes, after which the surviving virus was plaqued on MA104 cell culture by standard technique. Plaques induced in MA104 cell culture were harvested at random, propagated in MA104 cell culture, and analyzed by PAGE-SS for dsRNA electropherotype in comparison with parental rotaviruses WC3 and WI61.

Among these plaque isolates was a reassortant rotavirus, designated WI61-4, containing gene 4 (v.p. 4) from human serotype 9, WI61, and all other genes derived from bovine rotavirus strain WC3. Also identified in this manner was reassortant WI61-7,9 containing genes 7 and 9 (v.p. 7) from WI61 and remaining segments from WC3.

### D. WISC2-4

WISC2-4 reassortant, containing the v.p.4 of human serotype 2, rotavirus strain WISC2, is prepared essentially as described above for other reassortants. Serum free (PBS, washed three times) monolayers of MA104 or Vero cell culture in 24 well plates are co-injected with human type 2 rotavirus isolate WISC2 [isolated at the Wistar Institute, Philadelphia, PA in the laboratory of Dr. H F. Clerk] and type 1 v.p.4 WC3 reassortant WI79-4, described above. Cell culture wells are infected with WISC2 at input multiplicities of 10.0 to 0.01 and with the more rapidly replicating WI79-4 at multiplicities of 0.1 to 0.0001 and fed with serum-free Eagle's MEM containing 0.5 µg.ml trypsin. When cytopathic effect (CPE) involves ≥50% of cells in a given well, the culture is harvested, frozen and thawed, and a supernatant evaluated for the specificity of rotavirus gene replication by polyacrylamide electrophoresis (PAGE) as described in Dolan et al, J. Clin. Microbiol., 21:753 (1985).

Infected cultures equally co-infected with both parent rotaviruses are then plaque-titrated on MA104 or Vero cells as described in Offit et al, Inf. Immun., 42 : 293 (1983). Pretreatment with anti-serum to rotavirus type 1 (Wa) enriches the preparation for WISC2-4 reassortants by neutralizing the WI79-4 parent virus. Plaques are propagated to high titer in MA104 cells and are analyzed by PAGE.

### Exemple 3 - Method for Making Exemplary Vaccines

### A. WC3:2-5

WC3:2-5 reassortant was adapted to growth in CV-1 cell culture by four additional passages in CV-1 cells. The four CV-1 cell passage comprises a test vaccine evaluated in human adult volunteers and in infants. Vero cells may also be used.

The vaccine was produced by inoculation of 850 cm² plastic roller bottles of CV-1 cell culture grown in Eagle's MEM medium containing 10% fetal calf serum and 25 micrograms/ml gentamicin. The cells were inoculated when a confluent monolayer was obtained six days after cell seeding. The cell cultures were washed twice with PBS to remove residual serum and inoculated with 10 ml of WC3:2-5 rotavirus stock containing a total of 6.0 X 10⁶ pfu (multiplicity of infection MOI was approximately 0.10). The virus was allowed to absorb to the cells by incubation at 37°C for 60 minutes. The inoculum was removed and the cells were fed with 80 ml per roller flask of BHK medium serum-free and containing 5.0 microgram/ml of unpurified trypsin and 25 microgram/ml of gentamicin.

The rotavirus-infected cell cultures were incubated at 37°C until the entire monolayer exhibited CPE, approximately 72 hours. The virus was then harvested by disrupting the cells with three cycles of freezing and thawing. Cell debris was removed by centrifugation at 2000Xg for 60 minutes at 4°C. The resulting supernatant fluid comprised the test vaccine. It was frozen at -70°C pending testing for sterility, freedom from adventitious viruses, and assay of concentration of infectious reassortant rotavirus.

Sterility tests consisted of inoculation of the vaccine into standard laboratory media for the culture of aerobic and anaerobic bacteria, mycobacteria, and fungi. The vaccine was tested for mycoplasma by inoculation of 3T3 mouse cells in culture, followed by staining with Hoechst stain for intracytoplasmic DNA. Testing for adventitious viruses included inoculation of human and primate cell cultures in the presence of serotype-specific anti-rotavirus serum obtained by conventional methods, to suppress the replication of vaccine virus, which were observed for the appearance of CPE and/or hemadsorption. Adult and newborn mice were inoculated intracerebrally and orally with the vaccine and observed subsequently for 30 days. Adult guinea pigs were inoculated intraperitoneally and observed for 15 days post-inoculation.

Infectious reassortant rotavirus WC3:2-5 concentration was determined by standard plaque assay. The vaccine in this example had a titer of 10^{6.3} pfu/ml. The vaccine stock has been deposited with the American Type Culture Collection, as ATCC No. VR2195.

### B. WI79-9

Reassortant rotavirus WI79-9 was passaged a total of six times in MA104 cell culture which included three serial plaque purifications and then was adapted to growth in CV-1 culture by three passages in CV-1 cells. The third CV-1 cell passage represents a test vaccine evaluated in adult volunteers and infants. Vero cells may also be used.

The text vaccine was produced in a manner similar to that used for WC3:2-5 in part A, above. Roller bottles (850 cm²) of CV-1 cells were infected with WI79-9 reassortant rotavirus at a M.O.I. of approximately 0.30. Virus was adsorbed for thirty minutes at 37°C, after which the cell cultures were fed with 100 ml/roller bottle of BHK medium, serum-free, containing 25 micrograms/ml gentamicin and 1.0 microgram/ml purified trypsin (Sigma Chemical Company). Infected cell cultures were incubated at 37°C and harvested when CPE involved the entire monolayer at 72 hours post-infection. The methods for harvesting and clarifying the virus to produce the test WI79-9 vaccine and the methods used to ensure sterility and freedom from adventitious viruses were identical to those described in part A, above. WI79-9 reassortant rotavirus vaccine had an infectivity titer of 10^{7.5} pfu/ml. The vaccine stock has been deposited with the American Type Culture Collection, as ATCC No. VR2196.

### C. WISC2-4

WISC2-4 reassortant is adapted to growth in Vero cell culture by serial passage in roller tubes of Vero cells. When WISC2-4 is adapted to grow to high titer in Vero roller tubes, ≥10^{7.0} pfu/ml, a vaccine test lot is prepared in T150 flasks of Vero cells (300 to 1000 ml).

The proposed vaccine is tested for adventitious agents by inoculation onto standard bacteriological, fungal and mycoplasma media and primary primate cell cultures and by inoculation of newborn mice, adult mice and guinea pigs, as described above for WC3:2-5. Virus titer is determined by plaque assay in MA104 cells of 10% stool suspension according to the technique of Clark et al, as described in Am. J. Dis. Children, 140:350 (1986). Reassortant identity and purity is determined by PAGE.

### Example 4 - Administration of Vaccines

### A. WC3:2-5

Administration of vaccine to adults: A full dose (10^{6.3} pfu) of WC3:2-5 was administered orally to seven normal adult volunteers of age 31 to 50 years. Volunteers were given 30 ml of Maalox (Rorer) prior to vaccine, to buffer stomach acids. All remained clinically normal for 30 days. None was observed to be shedding vaccine rotavirus in feces three days after inoculation. One adult exhibited an increase in serum antibody to both WC3 rotavirus and to human serotype 3 rotavirus at 30 days post-inoculation. The other volunteers, who were all previously seropositive to rotavirus of serotype 1 and/or 3 and/or bovine serotype, did not exhibit a rise in serum antibody to serotype 3 or bovine serotype rotavirus. No volunteer exhibited a rise in antibody to serotype 1 rotavirus.

Administration of vaccine to infants: In a cautious series of vaccine trials, WC3:2-5 was first administered in a reduced dose of 10^{5.3} pfu to two infants who had been previously immunized with WC3 vaccine, then to two infants given WC3:2-5 vaccine as an original dose of 10^{4.3} pfu and then to two infants given an original dose of 10^{5.3} pfu. Finally, 24 infants were given a full dose of 10^{6.3} pfu. All infants were given vaccine orally in a dose of 2.5 ml containing 20% cherry syrup. In approximately one half of the infants stomach acids were neutralized by oral administration of at least 30 ml of infant formula, no more than 30 minutes prior to vaccine administration.

All infants were monitored for signs of vaccine-associated illness for the first seven days post-inoculation, but no symptoms were observed. A fecal sample was collected between three and seven days post inoculation for detection of vaccine rotavirus by standard plaque assay: a single infant shed vaccine rotavirus in low titer (less than 10^{3.0} pfu/gram feces) after receiving a vaccine dose of 10^{5.3} pfu. Twelve of 29 (41%) of the infants who completed the 30 day trial exhibited an increase in serum antibody at 30 days post infection when sera were tested against bovine rotavirus strain WC3 and prototype rotavirus strains SAll (serotype 3) and Wa (serotype 1). The observed serum antibody responses were most frequently directed towards the bovine serotype WC3 rotavirus (8 of the 29 infants, or 28%) and the serotype 3 rotavirus strain SAll (9 of the 29 infants, or 31%), reflecting the bivalent serotype 3 and bovine serotype constitution of the WC3:2-5 reassortant rotavirus. Only 4 infants, or 14% demonstrated SN response to Wa.

Analysis of the WC3:2-5 reassortant virus-vaccinated population (only the 24 infants given a full dose of 10^{6.3} pfu) indicated that infants 5 to 11 months old responded more efficiently (6/11, or 55%), than those 2 to 4 months old (3/13, or 23%). Infants with low titers of serum antibody prior to administration of vaccine were also more likely to exhibit an immune response to vaccine. For example, in the population of infants 5 to 11 months old possessing serum antibody titers of less than 1:250 to serotype 3 rotavirus prior to vaccine, 6 of 8, or 75%, developed a rise in serum antibody in response to vaccine.

The percentage of infants developing an immune response could also be enhanced by administration of a second dose of WC3:2-5 vaccine given orally 30 days after the original dose. Therefore, of 19 infants given two full doses, seven of nineteen, or 37%, responded to the first dose; 10 of 19, or 53%, responded to the second booster dose, and 13 of 19, or 68%, responded to either the first, second or both doses.

Antigenic variation between different rotavirus strains classified within serotype 3 has been observed, when compared in cross neutralization tests using hyperimmune [Hoshino et al, (1984)] or monoclonal [Taniguchi et al, (1985)] antisera. Therefore, the sera collected from 18 infants 30 days after their first dose of WC3:2-5 reassortant virus were tested for neutralization antibody to WC3:2-5 reassortant as well as to the SAll prototype serotype 3 rotavirus. Many infants developed an immune response to WC3:2-5 reassortant virus in the absence of a detectable serum antibody response to SAll virus. After a single dose of WC3:2-5 vaccine, the combined incidence of infants with a serum antibody response to either of the serotype 3 viruses was 13 of 18, or 72%.

### B. WI79-9

Administration of vaccine to adults: Four adult volunteers were given a full dose (10^{7.5} pfu) of WI79-9 vaccine orally after oral administration of 30 ml of Maalox to buffer stomach acids. All adults remained clinically normal. None shed vaccine rotavirus in stool samples collected three days post infection.

Administration of vaccine to infants: WI79-9 vaccine was administered orally to infants in a volume of 2.5 ml, including 2.0 ml of vaccine and 0.5 ml of cherry syrup. Infants were given 30 ml of infant formula, or occasionally 1 ml/kg body weight of Maalox 30 minutes prior to vaccine to buffer stomach acids. In sequence, two infants were given a WI79-9 dose of 10^{5.5} pfu; two were given a dose of 10^{6.5} pfu; and 49 infants and one three year old child were given a dose of 10^{7.5} pfu. No vaccine associated symptoms of disease were observed. Four of 50 infants given a full dose shed detectable levels of vaccine virus in stool. 30 of 54 infants, or 57%, given any dose of vaccine developed a virus-neutralizing serum antibody response to one or more of rotavirus serotypes 1, 3, or bovine. This immune response to a primary dose of WI79-9 was most often directed against the bovine serotype of rotavirus, WC3, or serotype 1, WI79, reflecting the bivalent antigenic constitution of the reassortant rotavirus.

The efficiency of induction of an immune response to WI79-9 in infants could be further enhanced by giving a second "booster" dose of vaccine orally, 30 days after the primary dose. Such a booster could consist of the WI79-9 reassortant virus used for the original inoculation or a vaccine consisting of either virus parent to the WI79-9 reassortant. The combined results with the WI79-9 virus vaccine followed by any of the three booster doses gave a 71% incidence of serum antibody response in 2 to 4 month old infants and 91% in 5 to 11 month old infants. Following a booster dose, heterotypic antibody to serotype 3 (SA11) rotavirus was also induced with a frequency similar to that obtained to bovine serotype or with serotype 1 rotavirus. Thus, antibody was induced to the two serotypes, 1 and 3, most often responsible for rotavirus disease in infants in the United States.

Additional studies of this vaccine are described in detail in H F. Clerk et al, J. Infect. Dis., 161:1099-1104 (1990) and H F. Clerk et al, Vaccine, 8:327-333 (1990). Both references are incorporated herein by reference.

### C. WISC2-4

Adult subjects are healthy volunteers over the age of 21; infants are aged 5-11 months. Five adults are given the full dose (10^{7.0} pfu). Two infants are given 10⁴ pfu. Two infants are given 10⁶ pfu and 20 infants are given the full dose. Between the first three adults and the first six infants given each vaccine, there is an interval of at least five days. Adult vaccinees provide a blood and fecal sample followed by administration of 1 ml/kg Maalox buffer, and immediately thereafter the vaccine, in 2.5 ml doses. Fecal and blood samples are taken followed by ca. 30 ml oral infant formula for buffering and 2.5 ml vaccine for infants.

Fecal samples are collected on day 5 after vaccination. On day 28 fecal and blood samples are collected, and a second vaccine dose is given. A final fecal sample is collected after 5 days and a final blood sample is collected 28 days after the second dose.

Vaccine virus shed in feces is determined by plaque assay in MA104 cells of 10% stool suspension. Selected virus plaques induced by 5 day post-vaccine stool samples are harvested, propagated in MA104 cells and evaluated for vaccine virus genotype by PAGE. All serum sample pre-vaccine and post-doses 1 and 2 are titrated to endpoint for plaque-neutralizing antibody to WC3 virus and to the rotavirus strain contributing the v.p. 4 antigen. All sera indicating positive response to vaccine or sero-positive pre vaccine are tested against types 1, 2, 3 and 4 rotavirus. A positive response is a 3-fold rise in serum neutralizing antibody.

Numerous modifications may be made by one skilled in the art to the methods and compositions of the present invention in view of the disclosure herein. Such modifications are believed to be encompassed in the appended claims.

## Claims (Claims for the following Contracting State(s): AT, BE, DE, FR, GB, IT, LU, NL, SE, CH, DK)

1. A rotavirus reassortant useful as a component of a vaccine against human rotavirus infection, said reassortant comprising gene 4 from a human rotavirus encoding the v.p.4 neutralization antigen and the gene from a human rotavirus encoding the v.p.7 neutralization antigen, said reassortant characterized by remaining rotavirus segments originating solely from a bovine WC3 strain rotavirus or progeny of said strain substantially identical thereto or from both said human and said bovine strains, and said reassortant further characterized as capable of inducing a protective immune response in human infant and child patients to challenge with native rotavirus without producing serious adverse effects in said patients.

2. The reassortant according to claim 1 wherein said human rotavirus is selected from the group consisting of virus serotype 1, serotype 2, serotype 3, serotype 4, serotype 8, and serotype 9.

3. The reassortant according to claim 2 wherein said human rotavirus strain contributing the v.p.7 encoding gene is identical to the rotavirus strain contributing the v.p.4 encoding gene.

4. The reassortant according to claim 3 wherein the v.p.7 encoding gene is gene 9 found in WI79-9 (ATCC-VR-2194 and ATCC-VR2196) and the rotavirus segments contributed by a bovine WC3 strain rotavirus or progeny are genes 1-3, 5-8 and 10-11 found in WI79-9 (ATCC-VR 2/94 and ATCC-VR 2196).

5. A vaccine for providing immunological protection against acute diarrhea caused by human rotavirus, said vaccine comprising at least one rotavirus reasscrtant comprising gene 4 from a human rotavirus encoding the v.p. 4 neutralization antigen and the gene from a human rotavirus encoding the v.p. 7 neutralization antigen, said reassortant characterized by remaining rotavirus segments originating solely from a bovine WC3 strain rotavirus or progeny of said strain substantially identical thereto or from both said human and said bovine stains and said reassortant further characterized as capable of inducing a protective immune response in human infant and child patients to challenge with native rotavirus without producing serious adverse effects in said patients.

6. A vaccine according to claim 5 in unit dose form comprising from about 10^{6.0} to about 10^{9.0} pfu suitable for administration to a human infant or child by the oral or nasal route or by injection.

7. A method for preparing a reassortant virus comprising examining, by polyacrylamide gel electrophoresis, progeny clones from plaques produced in a suitable cell substrate infected with a mixed infection of a bovine rotavirus strain WC3 and a human rotavirus, under conditions enabling gene reassortment therein, for the presence of a reassortant containing gene segments from both the bovine and human rotavirus, said reassortant containing the gene encoding the v.p. 4 antigen from the human rotavirus and the gene encoding the v.p. 7 antigen from the human rotavirus, said reassortant characterized by remaining rotavirus segments originating solely from a bovine WC3 strain rotavirus or progeny of said strain substantially identical thereto or from both said human and said bovine strains.

8. The method according to claim 7 wherein said cell substrate is selected from the group consisting of CV-1, Vero, BSC-1, MA104 and primary primate kidney cell cultures.

9. The vaccine according to claim 5 further comprising one or more additional reassortants.

10. The vaccine according to claim 9 comprising an additional rotavirus reassortant comprising from a human rotavirus either the gene 4 encoding the v.p.4 neutralization antigen or the gene encoding the v.p.7 neutralization antigen, the other of said two genes originating from a bovine WC3 strain rotavirus or progeny thereof substantially identical thereto and all the other rotavirus segments of the additional rotavirus reassortant originating either from said bovine WC3 strain rotavirus or progeny thereof or from both said bovine strain and said human strain.

11. The vaccine according to claim 9 wherein said additional reassortants comprise human v.p.4 gene segments of different human rotavirus serotypes.

12. The vaccine according to claim 9 wherein said additional reassortant consists of a bovine and human rotavirus reassortant consisting of the v.p.4 neutralization antigen from a human rotavirus, and all remaining rotavirus gene segments originating from the bovine strain rotavirus.

## Claims (Claims for the following Contracting State(s): ES, GR)

1. A method for preparing a rotavirus reassortant useful as a component of a vaccine against human rotavirus infection, said reassortant rotavirus comprising:
(a) gene 4 from a human rotavirus encoding the v.p.4 neutralization antigen,
(b) the gene from a human rotavirus encoding the v.p.7 neutralization antigen, and characterized by
(c) remaining rotavirus segments originating solely from a bovine WC3 strain rotavirus or progeny of said strain substantially identical thereto or from both said human and said bovine strains and said reassortant further characterized as capable of inducing a protective immune response in human infant and child patients to challenge with native rotavirus without producing serious adverse effects in said patients,
said method comprising the steps of:
(a) infecting a suitable cell substrate with a mixed infection of a bovine rotavirus strain WC3 and a human rotavirus under conditions enabling gene reassortment in said infected culture;
(b) examining, by polyacrylamide gel electrophoresis, progeny clones from plaques produced in said cell substrate for the presence of a reassortant containing a human rotavirus gene segment 4 encoding the v.p.4 neutralization antigen and a human rotavirus gene segment encoding the v.p.7 neutralization antigen, said reassortant characterized by remaining rotavirus segments originating solely from a bovine WC3 strain rotavirus or progeny of said strain substantially identical thereto or from both said human and said bovine strains.

2. The method according to claim 1 wherein said human rotavirus is selected from the group consisting of virus serotype 1, serotype 2, serotype 3, serotype 4, serotype 8 and serotype 9.

3. The method according to claim 2 wherein said human rotavirus strain contributing the v.p.7-encoding gene is identical to the rotavirus strain contributing the v.p.4-encoding gene.

4. The method according to claim 3 wherein the rotavirus reassortant has gene 9 found in WI79-9 (ATCC-VR 2194 and ATCC-VR 2196) as the v.p.7-encoding gene and genes 1-3, 5-8 and 10-11 found in WI79-9 (ATCC-VR 2194 and ATCC-VR 2196) as the genes contributed by a bovine WC3 strain rotavirus or progeny.

5. The method according to claim 1 wherein said cell substrate is selected from the group consisting of CV-1, Vero, BSC-1, MA104 and primary primate kidney cell cultures.

6. A method for preparing a reassortant virus comprising examining, by polyacrylamide gel electrophoresis, progeny clones from plaques produced in a suitable cell substrate infected with a mixed infection of a bovine rotavirus strain WC3 and a human rotavirus, under conditions enabling gene reassortment therein, for the presence of a reassortant containing gene segments from both the bovine and human rotavirus, said reassortant containing the gene encoding the v.p.4 antigen from the human rotavirus and the gene encoding the v.p.7 antigen from the human rotavirus, said reassortant characterized by remaining rotavirus segments originating solely from a bovine WC3 strain rotavirus or progeny of said strain substantially identical thereto or from both said human and said bovine strains.

7. A method of preparing a vaccine for providing immunological protection against acute diarrhea caused by human rotavirus, comprising preparing a reassortant rotavirus by a method according to any of claims 1 to 6 and incorporating it into a vaccine.

8. The method according to claim 7 wherein the vaccine further comprises one or more additional reassortants.

9. The method according to claim 8 wherein the vaccine comprises an additional rotavirus reassortant comprising from a human rotavirus either the gene 4 encoding the v.p.4 neutralization antigen or the gene encoding the v.p.7 neutralization antigen, the other of said two genes originating from a bovine WC3 strain rotavirus and all the reaming rotavirus segments of the additional rotavirus reassortant originating either from a bovine WC3 strain rotavirus or progeny thereof substantially identical thereto or from both said bovine strain and said human strain.

10. The method according to claim 8 wherein said additional reassortants comprise human v.p.4 gene segments of different human rotavirus serotypes.

11. The method according to claim 8 wherein said additional reassortant consists of a bovine and human rotavirus reassortant consisting of the v.p.4 neutralization antigen from a human rotavirus, and all remaining rotavirus gene segments originating from the bovine strain rotavirus.

## Patentansprüche (Patentansprüche für folgende(n) Vertragsstaat(en): AT, BE, DE, FR, GB, IT, LU, NL, SE, CH, DK)

1. Rotavirus-Reassortante, die als Bestandteil eines Vakzins gegen Infektion mit menschlichem Rotavirus verwendet werden kann, wobei diese Reassortante das für das v.p.4-Neutralisierungsantigen codierende Gen 4 aus einem menschlichen Rotavirus und das für das v.p.7-Neutralisierungsantigen codierende Gen eines menschlichen Rotavirus enthält, wobei diese Reassortante dadurch gekennzeichnet ist, daß die übrigen Rotavirussegmente ausschließlich aus einem Rinder-Rotavirusstamm WC3 oder Nachkommen dieses Stamms, die hiermit im wesentlichen identisch sind, oder sowohl aus diesem menschlichen Stamm sowie diesem Rinderstamm stammen, und wobei diese Reassortante weiterhin dadurch gekennzeichnet ist, das sie bei menschlichen Patienten im Kleinkindes- und Kindesalter auf Provokation mit nativem Rotavirus eine schützende Immunantwort induziert, ohne bei diesen Patienten schwere Nebenwirkungen hervorzurufen.

2. Reassortante nach Anspruch 1, wobei der menschliche Rotavirus aus der Gruppe Virus-Serotyp 1, -Serotyp 2, -Serotyp 3, -Serotyp 4, -Serotyp 8 und -Serotyp 9 ausgewählt ist.

3. Reassortante nach Anspruch 2, wobei der menschliche Rotavirusstamm der das für v.p.7 codierende Gen zur Verfügung stellt, mit dem Rotavirusstamm, der das für v.p.4 codierende Gen zur Verfügung stellt, identisch ist.

4. Reassortante nach Anspruch 3, wobei es sich bei dem für v.p.7 codierenden Gen um das in WI 79-9 (ATCC-VR 2194 und ATCC-VR 2196) vorliegende Gen 9 und bei den von einem Rinder-Rotavirusstamm WC3 oder dessen Nachkommenschaft zur Verfügung gestellten Rotavirussegmenten um die in WI 79-9 (ATCC-VR 2194 und ATCC-VR 2196) vorliegenden Gene 1-3, 5-8 und 10-11 handelt.

5. Vakzin zur Bereitstellung von Immunschutz gegen durch menschliches Rotavirus verursachte akute Diarrhöe, wobei dieses Vakzin mindestens eine Rotavirusreassortante mit Gen 4 aus einem menschlichen Rotavirus, das für das v.p.4-Neutralisierungsantigen codiert, und das Gen aus einem menschlichen Rotavirus, das für das v.p.7-Neutralisierungsantigen codiert enthält, wobei diese Reassortante dadurch gekennzeichnet ist, daß die übrigen Rotavirussegmente ausschließlich aus einem Rinder-Rotavirusstamm WC3 oder Nachkommen dieses Stamms, die hiermit im wesentlichen identisch sind, oder sowohl aus diesem menschlichen Stamm sowie diesem Rinderstamm stammen, und wobei diese Reassortante weiterhin dadurch gekennzeichnet ist, daß sie bei menschlichen Patienten im Kleinkindes-und Kindesalter auf Provokation mit nativem Rotavirus eine schützende Immunantwort induziert, ohne bei diesen Patienten schwere Nebenwirkungen hervorzurufen.

6. Vakzin nach Anspruch 5 in Einzeldosisform mit ungefähr 10^{6,0} bis ungefähr 10^{9,0} PBE, das sich zur Verabreichung an den Menschen im Kleinkindes- oder Kindesalter auf oralem oder nasalem Weg oder mittels Injektion eignet.

7. Verfahren zur Herstellung eines reassortanten Virus, bei dem man Nachkommenschaftsklone aus in einem geeigneten, mit einer Mischinfektion eines Rinder-Rotavirusstamms WC3 und einem menschlichen Rotavirus infizierten Zellsubstrat unter Bedingungen, die die Reassortierung von Genen gestatten, produzierten Plaques mittels Polyacrylamid-Gelelektrophorese auf das Vorhandensein einer Reassortante prüft, die Gensegmente sowohl aus dem Rinder-Rotavirus als auch dem menschlichen Rotavirus enthält, wobei diese Reassortante das für das v.p.4-Antigen codierende Gen aus menschlichem Rotavirus und das für das v.p.7-Antigen codierende Gen aus menschlichem Rotavirus enthält, wobei diese Reassortante dadurch gekennzeichnet ist, daß die übrigen Rotavirussegmente ausschließlich aus einem Rinder-Rotavirusstamm WC3 oder Nachkommen dieses Stamms, die hiermit im wesentlichen identisch sind, oder sowohl aus diesem menschlichen Stamm sowie diesem Rinderstamm stammen.

8. Verfahren nach Anspruch 7, wobei das Zellsubstrat aus der Gruppe CV-1-, Vero-, BSC-1-, MA104- und primären Primatennieren-Zellkulturen ausgewählt ist.

9. Vakzin nach Anspruch 5, das weiterhin eine oder mehrere zusätzliche Reassortanten enthält.

10. Vakzin nach Anspruch 9 mit einer weiteren Rotavirusreassortante, die entweder das für das v.p.4-Neutralisierungsantigen codierende Gen 4 oder das für das v.p.7-Neutralisierungsantigen codierende Gen aus einem menschlichen Rotavirus enthält, wobei das jeweils andere Gen aus einem Rinder-Rotavirusstamm WC3 oder dessen Nachkommenschaft, die hiermit im wesentlichen identisch ist, stammt, und alle anderen Rotavirusabschnitte der zusätzlichen Rotavirusreassortante entweder aus diesem Rinder-Rotavirusstamm WC3 oder dessen Nachkommenschaft oder sowohl aus dem Rinderstamm als auch dem menschlichen Stamm stammen.

11. Vakzin nach Anspruch 9, wobei diese zusätzlichen Reassortanten menschliche v.p.4-Gensegmente anderer menschlicher Rotavirus-Serotypen enthalten.

12. Vakzin nach Anspruch 9, wobei diese zusätzliche Reassortante aus einer Reassortante aus Rinder- und menschlichem Rotavirus, die aus dem v.p.4-Neutralisierungsantigen aus einem menschlichen Rotavirus besteht, wobei alle restlichen Rotavirusgensegmente aus dem Rinderrotavirusstamm stammen, besteht.

## Patentansprüche (Patentansprüche für folgende(n) Vertragsstaat(en): ES, GR)

1. Verfahren zur Herstellung einer Rotavirusreassortante, die als Bestandteil eines Vakzins gegen Infektion mit menschlichem Rotavirus verwendet werden kann, wobei dieses reassortante Rotavirus folgendes umfaßt:
(a) das für das v.p.4-Neutralisierungsantigen codierende Gen 4 aus einem menschlichen Rotavirus,
(b) das für das v.p.7-Neutralisierungsantigen codierende Gen aus einem menschlichen Rotavirus, dadurch gekennzeichnet, daß
(c) die übrigen Rotavirussegmente ausschließlich aus einem Rinder-Rotavirusstamm WC3 oder Nachkommen dieses Stamms, die hiermit im wesentlichen identisch sind, oder sowohl aus diesem menschlichen Stamm sowie diesem Rinderstamm stammen, und wobei diese Reassortante weiterhin dadurch gekennzeichnet ist, daß sie bei menschlichen Patienten im Kleinkindes- und Kindesalter auf Provokation mit nativem Rotavirus eine schützende Immunantwort induziert, ohne bei diesen Patienten schwere Nebenwirkungen hervorzurufen,
wobei dieses Verfahren folgende Schritte umfaßt:
(a) Infizieren eines geeigneten Zellsubstrats mit einer Mischinfektion aus einem Rinder-Rotavirusstamm WC3 und einem menschlichen Rotavirus unter Bedingungen, die die Genreassortierung in dieser infizierten Kultur gestatten;
(b) Prüfen von Nachkommenschaftsklonen von in diesem Zellsubstrat produzierten Plaques mittels Polyacrylamid-Gelelektrophorese auf das Vorhandensein einer Reassortante, die ein für das v.p.4-Neutralisierungsantigen codierendes Gensegment 4 aus einem menschlichen Rotavirus sowie ein für das v.p.7-Neutralisierungsantigen codierendes Gensegment eines menschlichen Rotavirus enthält, wobei diese Reassortante dadurch gekennzeichnet ist, daß die übrigen Rotavirussegmente ausschließlich aus einem Rinder-Rotavirusstamm WC3 oder Nachkommen dieses Stamms, die hiermit im wesentlichen identisch sind, oder sowohl aus diesem menschlichen Stamm sowie diesem Rinderstamm stammen.

2. Verfahren nach Anspruch 1, wobei der menschliche Rotavirus aus der Gruppe Virus-Serotyp 1, -Serotyp 2, -Serotyp 3, -Serotyp 4, -Serotyp 8 und -Serotyp 9 ausgewählt ist.

3. Verfahren nach Anspruch 2, wobei der menschliche Rotavirusstamm, der das für v.p.7 codierende Gen zur Verfügung stellt, mit dem Rotavirusstamm, der das für v.p.4 codierende Gen zur Verfügung stellt, identisch ist.

4. Verfahren nach Anspruch 3, wobei die Rotavirus -Reassortante als das für v.p.7 codierende Gen über das in WI79-9 (ATCC-VR 2194 und ATCC-VR 2196) vorliegende Gen 9 und als die von einem Rinderrotavirusstamm WC3 oder dessen Nachkommenschaft zur Verfügung gestellte Gene über die in WI79-9 (ATCC-VR 2194 und ATCC-VR 2196) vorliegenden Gene 1-3, 5-8 und 10-11 verfügt.

5. Verfahren nach Anspruch 1, wobei das Zellsubstrat aus der Gruppe CV-1-, Vero-, BSC-1-, MA104- und primären Primatennieren-Zellkulturen ausgewählt ist.

6. Verfahren zur Herstellung eines reassortanten Virus, bei dem man Nachkommenschaftsklone aus in einem geeigneten, mit einer Mischinfektion eines Rinder-Rotavirusstamms WC3 und einem menschlichen Rotavirus infizierten Zellsubstrat unter Bedingungen, die die Reassortierung von Genen gestatten, produzierten Plaques mittels Polyacrylamid-Gelelektrophorese auf das Vorhandensein einer Reassortante prüft, die Gensegmente sowohl aus dem Rinder-Rotavirus als auch dem menschlichen Rotavirus enthält, wobei diese Reassortante das für das v.p.4-Antigen codierende Gen aus menschlichem Rotavirus und das für das v.p.7-Antigen codierende Gen aus menschlichem Rotavirus enthält, wobei diese Reassortante dadurch gekennzeichnet ist, daß die übrigen Rotavirussegmente ausschließlich aus einem Rinder-Rotavirusstamm WC3 oder Nachkommen dieses Stamms, die hiermit im wesentlichen identisch sind, oder sowohl aus diesem menschlichen Stamm sowie diesem Rinderstamm stammen.

7. Verfahren zur Herstellung eines Vakzins, das gegen durch menschliches Rotavirus hervorgerufene akute Diarrhöe Immunschutz bereitstellt, bei dem man ein reassortantes Rotavirus durch ein Verfahren nach einem der Ansprüche 1 bis 6 herstellt und in einen Impfstoff integriert.

8. Verfahren nach Anspruch 7, wobei das Vakzin weiterhin einen oder mehrere zusätzliche Reassortanten enthält.

9. Verfahren nach Anspruch 8, wobei das Vakzin eine zusätzliche Rotavirusreassortante enthält, die entweder das für das v.p.4-Neutralisierungsantigen codierende Gen 4 oder das für das v.p.7-Neutralisierungsantigen codierende Gen aus einem menschlichen Rotavirus enthält, wobei das jeweils andere dieser Gene aus einem Rinder-Rotavirusstamm WC3 stammt und alle restlichen Rotavirussegmente der zusätzlichen Rotavirusreassortante entweder von einem Rinderrotavirusstamm WC3 oder dessen hiermit im wesentlichen identischen Nachkommenschaft oder sowohl aus diesem Rinderstamm als auch diesem menschlichen Stamm stammen.

10. Verfahren nach Anspruch 8, wobei diese zusätzlichen Reassortanten menschliche v.p.4-Gensegmente anderer menschlicher Rotavirus-Serotypen enthalten.

11. Verfahren nach Anspruch 8, wobei diese zusätzliche Reassortante aus einer Reassortante aus Rinder- und menschlichem Rotavirus, die aus dem v.p.4-Neutralisierungsantigen aus einem menschlichen Rotavirus besteht, wobei alle restlichen Rotavirusgensegmente aus dem Rinderrotavirusstamm stammen, besteht.

## Revendications (Revendications pour l'(les) Etat(s) contractant(s) suivant(s): AT, BE, DE, FR, GB, IT, LU, NL, SE, CH, DK)

1. Réassortant de rotavirus utile à titre de composant d'un vaccin contre l'infection à rotavirus humain, ledit réassortant comprenant le gène 4 d'un rotavirus humain codant pour l'antigène de neutralisation v.p.4 et le gène d'un rotavirus humain codant pour l'antigène de neutralisation v.p.7, ledit réassortant étant caractérisé par les segments de rotavirus restants ayant pour origine uniquement une souche WC3 de rotavirus bovin ou une progéniture de ladite souche sensiblement identique à celle-ci ou à la fois ladite souche humaine et ladite souche bovine, et ledit réassortant étant encore caractérisé comme étant susceptible d'induire une réponse immunitaire protectrice chez les malades en bas âge et enfants humains à une épreuve par le rotavirus natif sans produire d'effets néfastes graves chez lesdits malades.

2. Réassortant selon la revendication 1, caractérisé en ce que ledit rotavirus humain est choisi dans le groupe constitué du virus sérotype 1, sérotype 2, sérotype 3, sérotype 4, sérotype 8 et sérotype 9.

3. Réassortant selon la revendication 2, caractérisé en ce que ladite souche de rotavirus humain contribuant le gène v.p.7 est identique à la souche de rotavirus contribuant le gène codant pour v.p.4.

4. Réassortant selon la revendication 3, caractérisé en ce que le gène codant pour v.p.7 et le gène 9 présents dans WI 79-9 (ATCC-VR 2194 et ATCC-VR 2196) et les segments de rotavirus contribués par une souche WC3 de rotavirus bovin ou une progéniture sont les gènes 1-3, 5-8 et 10-11 présents dans WI 79-9 (ATCC-VR 2194 et ATCC-VR 2196).

5. Vaccin destiné à assurer une protection immunologique contre la diarrhée aiguë provoquée par le rotavirus humain, ledit vaccin comprenant au moins un réassortant de rotavirus comprenant le gène 4 d'un rotavirus humain codant pour l'antigène de neutralisation v.p.4 et le gène d'un rotavirus humain codant pour l'antigène de neutralisation v.p.7, ledit réassortant étant caractérisé par les segments de rotavirus restants ayant pour origine uniquement une souche WC3 de rotavirus bovin ou une progéniture de ladite souche sensiblement identique à celle-ci ou à la fois ladite souche humaine et ladite souche bovine, et ledit réassortant étant encore caractérisé comme étant susceptible d'induire une réponse immunitaire protectrice chez les malades en bas âge et enfants humains à une épreuve par le rotavirus natif sans produire d'effets néfastes graves chez lesdits malades.

6. Vaccin selon la revendication 5, sous forme de doses unitaires comprenant d'environ 10^{6,0} à environ 10^{9,0} ufp, adapté à l'administration à un enfant ou un enfant en bas âge humain par voie orale ou nasale ou par injection.

7. Procédé de préparation d'un réassortant de virus, comprenant l'examen, par électrophorèse sur gel de polyacrylamide, des clones de la progéniture provenant de plages produites dans un substrat cellulaire convenable infecté par une infection mixte par une souche WC3 de rotavirus bovin et un rotavirus humain, dans des conditions y permettant le réassortiment de gènes, pour la présence d'un réassortant contenant des segments de gène provenant aussi bien du rotavirus bovin que humain, ledit réassortant contenant le gène codant pour l'antigène v.p.4 du rotavirus humain et le gène codant pour l'antigène v.p.7 du rotavirus humain, ledit réassortant étant caractérisé par les segments de rotavirus restants ayant pour origine uniquement une souche WC3 de rotavirus bovin ou une progéniture de ladite souche sensiblement identique à celle-ci ou à la fois ladite souche humaine et ladite souche bovine.

8. Procédé selon la revendication 7, caractérisé en ce que ledit substrat cellulaire est choisi dans le groupe constitué de CV-1, de Vero, de BSC-1, de MA104 et de cultures primaires de cellules de rein de primates.

9. Vaccin selon la revendication 5, comprenant encore un ou plusieurs réassortants supplémentaires.

10. Vaccin selon la revendication 9, comprenant un réassortant de rotavirus supplémentaire comprenant, à partir d'un rotavirus humain, soit le gène 4 codant pour l'antigène de neutralisation v.p.4 soit le gène codant pour l'antigène de neutralisation v.p.7, l'autre desdits deux gènes ayant pour origine une souche VC3 de rotavirus bovin ou une progéniture de celle-ci sensiblement identique à celle-ci et l'ensemble des autres segments de rotavirus du réassortant de rotavirus supplémentaire ayant pour origine soit ladite souche WC3 de rotavirus bovin soit une progéniture de celle-ci soit à la fois ladite souche bovine et ladite souche humaine.

11. Vaccin selon la revendication 9, caractérisé en ce que lesdits réassortants supplémentaires comprennent des segments de gènes humains de v.p.4 de différents sérotypes de rotavirus humains.

12. Vaccin selon la revendication 9, caractérisé en ce que ledit réas sortant supplémentaire est constitué d'un réassortant de rotavirus bovin et humain constitué de l'antigène de neutralisation v.p.4 provenant d'un rotavirus humain, et l'ensemble des segments de gène de rotavirus restants ayant pour origine le rotavirus de souche bovine.

## Revendications (Revendications pour l'(les) Etat(s) contractant(s) suivant(s): ES, GR)

1. Procédé de préparation d'un réassortant de rotavirus utile à titre de composant d'un vaccin contre l'infection à rotavirus humain, ledit réassortant de rotavirus comprenant:
(a) le gène 4 provenant d'un rotavirus humain codant pour l'antigène de neutralisation v.p.4
(b) le gène provenant d'un rotavirus humain codant pour l'antigène de neutralisation v.p.7, et caractérisé par
(c) les segments de rotavirus restants ayant pour origine uniquement une souche WC3 de rotavirus bovin ou une progéniture de ladite souche sensiblement identique à celle-ci ou à la fois ladite souche humaine et ladite souche bovine, et ledit réassortant étant encore caractérisé comme étant susceptible d'induire une réponse immunitaire protectrice chez les malades en bas âge et enfants humains à une épreuve par le rotavirus natif sans produire d'effets néfastes graves chez lesdits malades,
ledit procédé comprenant les étapes consistant à :
(a) infecter un substrat cellulaire convenable par une infection mixte par une souche WC3 de rotavirus bovin et un rotavirus humain dans des conditions permettant le réassortiment de gènes dans ladite culture infectée;
(b) examiner, par électrophorèse sur gel de polyacrylamide, les clones de la progéniture provenant de plages produites dans ledit substrat cellulaire pour la présence d'un réassortant contenant un segment de gène 4 de rotavirus humain codant pour l'antigène de neutralisation v.p.4 et un segment de gène de rotavirus humain codant pour l'antigène de neutralisation v.p.7, ledit réassortant étant caractérisé par les segments de rotavirus restants ayant pour origine uniquement une souche WC3 de rotavirus bovin ou une progéniture de ladite souche sensiblement identique à celle-ci ou à la fois ladite souche humaine et ladite souche bovine.

2. Procédé selon la revendication 1, caractérisé en ce que ledit rotavirus humain est choisi dans le groupe constitué du virus sérotype 1, sérotype 2, sérotype 3, sérotype 4, sérotype 8 et sérotype 9.

3. Procédé selon la revendication 2, caractérisé en ce que ladite souche de rotavirus humain contribuant le gène codant v.p.7 est identique à la souche de rotavirus contribuant pour gène codant pour v.p.4.

4. Procédé selon la revendication 3, caractérisé en ce que le réassortant de rotavirus possède le gène 9 présent dans WI 79-9 (ATCC-VR 2194 et ATCC-VR 2196) à titre de gène codant pour v.p.7 et les gènes 1-3, 5-8, et 10-11 présents dans WI 79-9 (ATCC-VR 2194 et ATCC-VR 2196) à tire de gènes contribués par une souche WC3 de rotavirus bovin ou la progéniture.

5. Procédé selon la revendication 1, caractérisé en ce que ledit substrat cellulaire est choisi dans le groupe constitué de CV-1, de Vero, de BSC-1, de MA104 et de cultures primaires de cellules de rein de primates.

6. Procédé de préparation d'un réassortant de virus, comprenant l'examen, par électrophorèse sur gel de polyacrylamide, des clones de la progéniture provenant de plages produites dans un substrat cellulaire convenable infecté par une infection mixte par une souche WC3 de rotavirus bovin et un rotavirus humain, dans des conditions y permettant le réassortiment de gènes, pour la présence d'un réassortant contenant des segments de gène provenant aussi bien du rotavirus bovin que humain, ledit réassortant contenant le gène codant pour l'antigène v.p.4 du rotavirus humain et le gène codant pour l'antigène v.p.7 du rotavirus humain, ledit réassortant étant caractérisé par les segments de rotavirus restants ayant pour origine uniquement une souche WC3 de rotavirus bovin ou une progéniture de ladite souche sensiblement identique à celle-ci ou à la fois ladite souche humaine et ladite souche bovine.

7. Procédé de préparation d'un vaccin destiné à assurer une protection immunologique contre la diarrhée aiguë provoquée par le rotavirus humain, comprenant la préparation d'un réassortant de rotavirus par un procédé selon l'une quelconque des revendications 1 à 6 et son incorporation dans un vaccin.

8. Procédé selon la revendication 7, caractérisé en ce que le vaccin comprend encore un ou plusieurs réassortants supplémentaires.

9. Procédé selon la revendication 8, caractérisé en ce que le vaccin comprend un réassortant de rotavirus supplémentaire comprenant, à partir d'un rotavirus humain, soit le gène 4 codant pour l'antigène de neutralisation v.p.4 soit le gène codant pour l'antigène de neutralisation v.p.7, l'autre desdits deux gènes ayant pour origine une souche VC3 de rotavirus bovin et l'ensemble des segments de rotavirus restants du réassortant de rotavirus supplémentaire ayant pour origine soit ladite souche WC3 de rotavirus bovin soit une progéniture de celle-ci sensiblement identique à celle-ci soit à la fois ladite souche bovine et ladite souche humaine.

10. Procédé selon la revendication 8, caractérisé en ce que lesdits réassortants supplémentaires comprennent des segments de gènes humains de v.p.4 de différents sérotypes de rotavirus humains.

11. Procédé selon la revendication 8, caractérisé en ce que ledit réassortant supplémentaire est constitué d'un réassortant de rotavirus bovin et humain constitué de l'antigène de neutralisation v.p.4 provenant d'un rotavirus humain, et l'ensemble des segments de gène de rotavirus restants ayant pour origine le rotavirus de souche bovine.
